# EUROPEAN PATENT SPECIFICATION

(11) **EP 2 834 217 B1**
(45) Date of publication and mention of the grant of the patent: **04.07.2018**
(21) Application number: 13714288.1
(22) Date of filing: 04.04.2013
(51) Int. Cl.: C07D 213/82, B01J 2/04

(54) **NICOTINAMIDE POWDER AND PROCESS AND DEVICE FOR ITS PRODUCTION**
NICOTINAMIDPULVER UND VERFAHREN UND VORRICHTUNG ZU SEINER HERSTELLUNG
POUDRE DE NICOTINAMIDE ET PROCÉDÉ ET DISPOSITIF POUR SA FABRICATION

(30) Priority: 04.04.2012 EP 12163114
(43) Date of publication of application: 11.02.2015
(73) Proprietor: Lonza Ltd, 3930 Visp (CH)
(72) Inventor: GERRITZEN, Detlef, 3934 Zeneggen (CH); CLAUSEN, Norbert, 3968 Veyras (CH); IRLE, Heike, 3931 Lalden (CH); ZACHER, Uwe, Brig 3900 (CH)
(86) International application number: PCT/EP2013/057082
(87) International publication number: WO 2013/150090

(56) References cited:
- GB-A- 2 050 190
- US-A- 2 752 355
- US-B1- 6 711 831

## Description

The invention relates a nicotinamide powder, a process for its production, and devices and uses relating to the preparation of the nicotinamide powder.

### Background of the invention

Nicotinamide (niacinamide; nicotinic acid amide, NSA, CAS Nr. 98-92-0) is the amide of nicotinic acid (vitamin B3). It is a water-soluble vitamin and part of the vitamin B group. As such, it fulfills an important function in the human and animal body by virtue of being a part of the coenzymes NAD⁺ und NADP⁺. Nicotinamide deficiency in humans leads to various symptoms, including weight loss, memory disruption, sleep disorders and the skin disease pellagra, all of which can be treated by administering nicotinamide.

Nicotinamide is used as a food additive and pharmaceutical. It is usually applied as part of mixed compositions with other components, especially other vitamins. A common dosage form of nicotinamide or compositions comprising nicotinamide is a tablet. For such applications, a premix is usually produced in a first step, which is a mixture of nicotinamide with other vitamins and inactive ingredients, such as processing aids. The premix is then pressed into tablets.

It is desirable to produce such tablets by direct compression from the premix. In the direct compression method of tablet production, dry ingredients are thoroughly mixed and then compressed into tablets. This eliminates the drying steps associated with the wet granulation method. It also reduces the higher costs involved in wet granulation including increased equipment, labor, time, process validation and energy expenditure. However, direct compression requires special properties of the nicotinamide powder or powder composition comprising nicotinamide.

Direct compression is only applicable when a stable tablet is obtained. For direct compression, the nicotinamide powder should be pelletized with a relatively low force, whilst the resulting tablet should have a relatively high breaking force.

In industrial tablet production, various properties are required for good and stable workability. Amongst these, it is important that the nicotinamide powder has a good flowability. Flowability is important for various applications, such as packaging, transporting, storing, and mixing of such powders. Flowability is also essential for producing tablets from powders with industrial pelletizing machinery.

Further, flowability should be preserved when storing the powder. Nicotinamide powders known in the art tend to caking, lumping and agglomeration. Thereby, the flow into a pelletizing device is constricted, deposits in the machinery may form and the production capacity is reduced. Flowability is usually high, if the water content of the particles is low and caking is avoided.

The powder particles should have a high homogeneity. For example, this is important for uniform mixing and uniform absorption by an individual after consumption. Further, it is advantageous for workability if the powder does not comprise fine particles, which cause dusting.

Overall, a nicotinamide powder would be required having a good flowability and low caking tendency as well as good direct compressibility. At least in part, these properties are antipodal, because high flowability and low caking usually require high powder dryness, whereas compressibility is usually fostered by a certain moisture content. Therefore, nicotinamide powder known in the art do not combine high flowability and low caking with sufficient compressibility in direct compression. Commonly, if such powders have good flowability, they are not suitable for direct compression due to low tablet strength.

Various routes for the synthesis of nicotinamide are known in the art. The most common route is the hydrolysis of 3-cyanopyridine via chemical or enzymatic catalysis. The known synthesis methods have in common, that a solution of nicotinamide is obtained. Various methods have been described for obtaining nicotinamide powders from such solutions.

A common process for recovery of solid nicotinamide from aqueous solution is crystallization, followed by solid-liquid separation, as disclosed in DE 30 14 1 60 and GB 2050190 A. Solid nicotinamide is obtained in the form of needle shaped crystals, the powders having irregular particle size distribution, tending to dust formation and having a poor flowability. The suitability of nicotinamide produced in this way for direct compression is insufficient.

Another known process for processing such crystals comprises compacting and pelletizing solid crystalline nicotinamide by roll compaction and classification. Main disadvantages of this procedure are suboptimal properties of the resulting powder granulates and the need of further process steps.

JP 03-157131 A and CN 101569840 A disclose processes for obtaining nicotinamide powders, in which solid nicotinamide is melted and spray dried. A drawback of this process is thermal decomposition of nicotinamide in the hot product melt having a melting point of 131 °C. Further, although the dry products usually have good flowability, direct compression is not feasible because of low tablet strength.

Thus, there is a need for nicotinamide powders and processes for their production which overcome the above-mentioned problems. Specifically, there is a need for nicotinamide powder having high flowability and low caking tendency, which can be compressed directly, easily and efficiently into tablets having high strength.

### Problem underlying the invention

The problem underlying the invention is to provide a nicotinamide powder, processes for its production, which overcome the above-mentioned problems.

Specifically, a nicotinamide powder shall be provided, which has a good flowability, low caking tendency and which can be directly compressed and pelletized, thereby yielding tablets of high stability. The particles shall be highly homogeneous, have a uniform size distribution and regular shapes. The amount of dust in the powder shall be low.

The process shall be carried out continuously at an industrial scale. The process shall produce nicotinamide at a high yield and be environmentally friendly, especially require as little energy as possible and avoid waste production.

### Disclosure of the invention

Surprisingly, the problem underlying the invention is solved by the nicotinamide powder, and processes according to the claims. Further inventive embodiments are disclosed throughout the description.

Subject of the invention is a process for the preparation of a nicotinamide powder, comprising the steps of
(a) providing an aqueous solution of nicotinamide and
(b) spray drying the aqueous solution.

By the inventive process, a solid nicotinamide powder is obtained. The process of the invention is also a process for drying nicotinamide and/or a process for the preparation of a dry nicotinamide powder.

Spray drying is characterized by formation of solid powder particles from liquids in a single step of combined spraying and drying. The solution is heated and dispersed through a spray nozzle, usually an atomizer. Fine droplets dispersed from the nozzle are immediately subjected to drying. They are sprayed into a drying chamber, wherein they contact a heated gas stream. Usually, the drying chamber is positioned below the atomizers. The solvent is rapidly withdrawn from the droplets in the hot gas stream and solid particles are formed. Often, solvent removal is so rapid that the size and shape of the remaining solid particles resembles the size and shape of the droplets. The size, shape and residual moisture content of the solid particles can be controlled by various parameters, such as the temperatures of the solution and gas stream and the atomizer dimensions. Spray drying is distinct from other spraying processes, in which liquid solutions are simply sprayed.

In the inventive process, preferably all the nicotinamide is spray dried. It is not necessary to introduce additional aqueous nicotinamide into the process by other means, for example by spraying additional nicotinamide solution for supporting granulation. It is also not necessary to add any other liquids for supporting granulation. Thus the overall water consumption and energy required for water evaporation can be kept low.

After spray drying, preferably a thermal treatment of the product is carried out. In a preferred embodiment of the invention, the process comprises the additional step of
(c) fluidizing the spray-dried nicotinamide in a fluidized bed.

In a fluidized bed, a solid particulate substance is placed under conditions to cause the solid to behave like a fluid. This is achieved by a fluidizing gas stream, which passes through the particulate medium. As a result, the particulate medium has properties and characteristics of normal fluids, such as the ability to flow freely under the effect of gravity, or to be pumped using fluid technologies.

In the preferred process, the nicotinamide powder obtained from the spray drying step is fluidized. The fluidized bed dries the spray dried nicotinamide particles. More importantly, it supports agglomeration of the spray dried nicotinamide particles. Low weight particles are blown out of the fluidized bed by the fluidizing gas stream. They are released from the fluidized bed and re-enter the spray drying chamber, where they agglomerate with droplets released from the spray dryer or with other solid particles of sufficient moisture. Agglomeration of particles may also occur within the fluidized bed. Overall, only agglomerates having a minimum size and weight remain in the fluidized bed. The fluidizing gas stream also ensures that the agglomerates are not large lumps. According to the invention, it was found that when combining spray drying with fluidization of the product in a fluidized bed, the resulting powder has a good flowability and direct compressibility, but a relatively low tendency towards caking.

In a preferred embodiment of the invention, steps (b) and (c) are carried out in a fluidized spray dryer. In a preferred embodiment of the invention, the fluidized spray dryer comprises
(A) a central drying chamber,
(B) spray drying means positioned above the central drying chamber,
(C) a fluidized bed positioned below the central drying chamber, and
(D) means for withdrawing nicotinamide powder positioned below the central drying chamber.

A fluidized spray dryer combines means for spray drying with a fluidized bed (an internal fluidized bed) in a single apparatus. The fluidized spray dryer comprises a central drying chamber, in which the sprayed droplets and emerging solid particles are dried in a hot gas stream. The fluidized bed is positioned below the spray dryer. Solid particles produced by spray drying descend and enter the fluidized bed. The gas stream for operating the fluidized bed transfers dust and light particles out of the fluidized bed and reintroduces them into the drying chamber. Thereby, only particles having a weight within a defined range remain within the fluidized bed. Dust and light particles released from the fluidized bed mix with the spray dried droplets and moist particles and form agglomerates. Overall, only agglomerates having a certain size and weight are maintained in the fluidized bed. The agglomerates are dried in the fluidized bed. The average residence time in the fluidized bed is adjusted such that particles having a desired moisture content are obtained.

Preferably, the shape of the drying chamber supports particle sedimentation downwards into the fluidized bed. Preferably, the lower part of the drying chamber is conical to support the sliding of particles downwards into the fluidized bed. The upper part of the drying chamber may be cylindrical.

Solid nicotinamide powder is withdrawn from the fluidized spray dryer, preferably at a position below the central drying chamber. The means for withdrawal are typically at the attached to the fluidized spray dryer. Preferably, the product is withdrawn by an overflow from the fluidized bed. The fluidized particles withdrawn may still have a low residual water content, for example in the range of 0.05% to 1% (w/w).

In principle, fluidized spray dryers are known in the art. For example, a fluidized spray dryer applicable in the inventive process is described in WO 00/74835. Fluidized spray dryers are commercially available from the Niro Group, U.S., under the trademark FSD.

In preferred embodiments of the invention, the aqueous solution in step (a) comprises 40% to 90%, preferably 60% to 90%, more preferably 75% to 85% (w/w) nicotinamide. Preferably, the nicotinamide in the solution is pure or substantially pure, i.e. it does not comprise substantial amounts of other solid ingredients. Preferably, the amount of nicotinamide in the solution is more than 95%, preferably more than 99% or more than 99.9% (w/w), based on the total amount of all solids. In a preferred embodiment of the invention, the nicotinamide solution is a reaction product of a preceding process, in which nicotinamide is produced by organic synthesis, preferably from 3-cyanopyridine. In a preferred embodiment, the reaction product is concentrated before the inventive process, for example by water evaporation.

Preferably, the aqueous solution is heated before spraying. For example, the aqueous solution may be heated to a temperature between 50 °C and 100 °C, preferably between 75 °C and 95 °C or between 70 °C and 90 °C. When a high concentration of nicotinamide is desired, the temperature should be sufficiently high. For example, for a solution of 80% (w/w) nicotinamide in water, the temperature should be at least 80 °C.

Spraying is preferably conducted with an atomizer or multiple atomizers. Atomizers comprise a narrow section, in which a pressure drop is created. Preferably, the atomizer is a single-substance pressure atomizer. However, other atomizer devices known in the art may be used, such as rotary atomizers, pneumatic atomizers or propellant atomizers. Spray drying is conducted under pressure, for example between 0.2 MPa (2 bar) and 15 MPa (150 bar), preferably between 1.5 MPa (15 bar) and 5 MPa (50 bar). Atomizers and their use are described for example in Peter Walzel, "Spraying and Atomizing of Liquids", Ullmann's Encyclopedia of Industrial Chemistry, Wiley-VCH, 2010, DOI: 10.1002/14356007.b02_06.pub2.

Droplets emerging from the atomizers are dried in a gas stream. The spray drying atomizers are positioned in the vicinity of the gas stream. According to the invention, the gas stream is preferably a cocurrent gas stream, which flows in the same direction as the spray dried product. The gas stream hits the spray cloud released from the atomizers. When contacting the hot gas stream, the droplets evaporate moisture rapidly because of the overall vast surface areas of the small droplets. The gas is preferably air, which is preferably dry. Preliminary drying of the gas may be carried out by condensation.

Preferably, the gas stream for spray drying, preferably the cocurrent gas stream, is heated to a temperature between 100 °C and 180 °C, more preferably between 100 °C and 150 °C. The water content of droplets leaving the spray dryer is reduced rapidly upon entry into the drying chamber. After leaving the spray dryer, the particles in the process of drying and the droplets agglomerate with each other, or agglomerate with dust particles or low weight particles in the drying chamber. Dried and agglomerated particles descend towards the lower part of the drying chamber by gravity and enter the fluidized bed. Since particles produced in the process are agglomerates of multiple smaller particles, the shapes are approximately spherical. Irregular shapes with sharp edges or elongated structures are generally not obtained.

The temperature of the fluidized bed is controlled at least partially by the fluidizing gas stream. The gas is preferably air, which is preferably dried. The fluidizing gas stream and the fluidized bed preferably cool the spray dried particles. Preferably, the fluidizing gas stream has a temperature which is lower than the temperature of the gas stream for spray drying and/ or of the drying chamber. Preferably, the temperature difference is at least 20 °C or at least 50 °C. In a preferred embodiment of the invention, the fluidizing gas stream has a temperature between 30 °C and 90 °C, preferably between 40 °C and 70 °C.

In a preferred embodiment of the inventive process, the fine dust is removed from the powder. The fine dust fraction may especially comprise particles having a diameter below 75 µm, below 50 µm or below 25 µm. It is not desirable to obtain a fine dust fraction in the product. Fine dust reduces homogeneity and workability and is disadvantageous for many applications.

Preferably, nicotinamide fine dust is withdrawn from the fluidized spray dryer, the nicotinamide fine dust is then accumulated in accumulation means, which preferably comprise at least one cyclone and/or filter, and nicotinamide fine dust thus accumulated is reintroduced into the fluidized spray dryer. The dust is reintroduced into the drying chamber, where it can agglomerate with other particles and droplets released from the spray dryer. When proceeding accordingly, the nicotinamide dust is recycled and integrated into the nicotinamide product. Preferably, the fine dust is withdrawn together with discharged air. The nicotinamide fine dust may be withdrawn from the fluidized spray dryer with discharged air at a position which is relatively high, such that larger agglomerates are not withdrawn. Preferably, the temperature of the discharged air is not too high, such that dust particles are not dried totally, but preserve a residual moisture content. For example, the temperature of the air discharged from the fluidized spray dryer may be below 90 °C, or between 60 and 90 °C. According to the invention, it was found that when maintaining a residual moisture content in the dust, subsequent agglomeration of the fine dust in the drying chamber can be improved.

Methods and means for accumulating dusts are known in the art. In a preferred embodiment, the dust is accumulated (pulverized) in a cyclone, or a combination of two or more cyclones in series. A cyclone recovers solid particles from a gas by vortex separation using rotational effects and gravity. In another preferred embodiment, the dust is accumulated with at least one filter, such as a bag filter. Filters are advantageous for recovering even very fine dust particles. The use of cyclones and/or filters is advantageous, because the dust is accumulated in pulverized form for further use. Pulverization of the dust prevents sticking to recycling devices and connections. The pulverized nicotinamide is re-introduced into the fluidized spray, where it can agglomerate with other particles. Overall, the nicotinamide dust is recycled. The fine dust can be recovered almost quantitatively and incorporated into the product, such that a high yield of nicotinamide is achieved. A final product is obtainable, which does not comprise fine dust or at least not significant amounts and thus has a good workability.

Preferably, the agglomerated particles are continuously withdrawn from the fluidized bed. Preferably, particles are removed from the fluidized bed by an overflow. The amount removed and/or time point may be controlled by a pressure drop of the fluidized bed indicating that the amount of particles in the bed has reached a critical value. For example, if a critical value is reached, an outlet could automatically be opened for release of particles.

The residence time of the particles in the fluidized spray dryer and in the fluidized bed shall be sufficiently high to ensure homogeneous product properties. For example, the average residence time of the particles in the fluidized bed sprayer may be between 1 min and 1 hour, preferably between 2 and 20 min.

Overall, agglomerated particles having a defined size and weight distribution can be produced with the fluidized spray dryer. Thereby, it is possible to obtain a highly uniform particle size distribution. According to the invention, it was found that a uniform powder is obtainable from the fluidized spray dryer having excellent flowability, excellent direct compressibility and a low tendency to caking.

Solid nicotinamide powder released from the fluidized spray dryer may comprise residual moisture. In a preferred embodiment of the invention, a nicotinamide powder obtained from the fluidized spray dryer is subjected to an additional drying step. This additional drying step is preferably carried out by an external device, which is not an internal component of the fluidized spray dryer. According to the invention, the water content of the articles is decreased further by the additional drying step. Thereby, the tendency of the solid product to agglomeration and caking can be decreased further. However, the additional drying step is optional. Nicotinamide powder released from the fluidized spray dryer already has advantageous properties and sufficient dryness for many applications.

In a preferred embodiment of the invention, the additional drying step is conducted with at least one external fluidized bed. The additional fluidized bed dries the powder further, i. e. the water content is reduced further. It also enhances homogeneity of the product and provides low granular abrasion. According to the invention, it was found that an additional external fluidized bed treatment decreases the caking tendency of the product further. Preferably, the external fluidized bed comprises a fluidizing gas stream having a temperature between 50 °C and 100 °C. The gas is preferably dry air. When maintaining the particles in the fluidized bed for a defined time range, uniformity of the product is increased. The average residence time in the external fluidized bed is preferably between 30 min and 6 hours, more preferably between 2 and 4 hours.

In a preferred embodiment of the invention, two or more external fluidized beds are used, for example 2, 3, 4, 5, 6 or more external fluidized beds, through which the powder is passaged. Thus the multiple fluidized beds are arranged in series. Alternatively, an external fluidized bed may comprise multiple sections. When passaging the particles through multiple fluidized beds, the average residence time of the particles is harmonized. Thus the uniformity of the product is improved, for example with respect to moisture content and internal moisture distribution.

In a preferred embodiment, at least two external fluidized beds are used having different temperatures. In this embodiment, one or more fluidized beds have a higher temperature for drying of the particles, whereas another fluidized bed has a lower temperature for cooling the particles. Preferably, the temperature for drying is between 50 °C and 120 °C, preferably between 60 °C and 100 °C. Preferably, the temperature for cooling is between 0 °C and 50 °C, more preferably between 5 °C and 25 °C. The cooling step is carried out after the drying step and adjusts the powder temperature for subsequent applications, such as packing.

In a preferred embodiment, more than two fluidized beds are used for drying and one subsequent fluidized bed is used for cooling. Preferably, three consecutive fluidized beds operated at a temperature between 60 °C and 100 °C and one subsequent fluidized bed operated at a temperature between 5 °C and 25 °C are used. In this arrangement, the additional external drying means are a series of four consecutive fluidized beds.

When using multiple external fluidized beds, the overall residence time can be adapted, and it can be ensured that all particles have been subjected to a sufficient drying treatment. The average residence time in the external fluidized beds is preferably between 30 min and 6 hours, more preferably between 2 and 4 hours.

In a preferred embodiment, fine dust is discharged from the external fluidized bed, collected and re-introduced into the process. Preferably, it is re-introduced into the fluidized spray dryer. Before re-introduction, it may be combined with another nicotinamide fine dust fraction of the process and/or accumulated in accumulation means, especially cyclones and/or filters. Preferably, the dine dust recovery means of the fluidized spray dryer and the external fluidized bed are merged.

In a preferred embodiment, the overall process is a continuous process. In other words, the introduction of the feed and gas streams, operation of the fluidized beds and withdrawal of the product are carried out substantially continuously. In a continuous process, the conditions in the fluidized spray dryer are essentially in equilibrium.

The product obtained from the overall process is preferably cooled. It may be sieved or subjected to other finishing steps. The product may then be packed, portioned etc.

According to the invention, a dry nicotinamide powder is obtained. The water content of the product obtained directly from the fluidized spray dryer may be below 1% (w/w) or below 0.5% (w/w), for example between 0.01% and 1% (w/w) or between 0.02% and 0.4% (w/w). When carrying out an additional drying step, for example with additional fluidized bed(s), the water content may be reduced further, for example for more than 10% or more than 50% (based on the water content before the additional drying step). The absolute water content of the nicotinamide powder may then be below 0.2% or below 0.1% (w/w). In a preferred embodiment of the invention, the moisture content of the nicotinamide powder obtained after the additional drying step, especially the additional fluidized bed(s) is between 0.005% and 0.2% (w/w), specifically between 0.01% and 0.1%. A residual water content within these ranges may be advantageous for the combination of good flowability, low caking and good direct compressibility.

Subject of the invention is also a nicotinamide powder, obtainable by the inventive process. Surprisingly, it was found that nicotinamide powder produced according to the inventive process has unique properties, which were not achieved by nicotinamide powders known in the art. On the one hand, the inventive product is dry, has a good flowability and does not tend to caking and lumping. Thus, it can be conveniently packed, transported and stored and has a good workability. On the other hand, the powder is suitable for direct compression and pelletizing, thereby obtaining tablets and pellets of high strength. This was unexpected, because commonly dry powders having a good flowability usually are not suitable for direct compression due to low moisture content. Without being bound to theory, the moisture distribution and internal structure of inventive powder particles may favor internal bonding strength within tablets or pellets.

Subject of the invention is also a nicotinamide powder, having
- an average particle diameter between 50 µm and 500 µm,
- an angle of repose below 40°, and
- a tablet breaking force above 150 N, preferably above 175 N, after direct compression of 330 mg nicotinamide into a standard convex tablet, diameter 9 mm, with a press force of 10 kN and/or a tensile strength of at least 2.5 N/mm², more preferably at least 3.0 N/mm², for a tablet obtained by direct compression with a compression pressure of 157 N/mm².

Tablet properties may be determined as outlined in the examples.

Preferably, the tensile strength is at least 2.5 N/mm², more preferably at least 3.0 N/mm², for a tablet obtained by direct compression with a compression pressure of 157 N/mm². Preferably, the tablet is produced as outlined in the examples. Tensile strength can be determined according to Jeckel, P. S., "Bestimmung wesentlicher Tabletteneigenschaften mit Hilfe der Nahinfrarot-Spektroskopie", Dissertation 2008, Universität Bonn.

Preferably, the Jenike flow function is above 30, above 40 or above 50. Preferably, Jenike flow function after time consolidation 24 h, 20 °C, at equilibrium relative humidity (ERH) is at least 8, more preferably at least 10. Jenike flow function may be determined according to standard method D6128 (ASTM, 2000), or as outlined in the examples.

Preferably, the bulk density of the powder is between 0.4 and 0.6 g/cm³, more preferably between 0.45 and 0.55 g/cm³. The average particle diameter may be between 50 µm and 500 µm, preferably between 80 µm and 250 µm.

The properties of the nicotinamide powder, such as dryness, particle size and particle size distribution, can be controlled by the inventive process. The properties are influenced by various parameters. For example, the nicotinamide concentration in the aqueous solution has an influence on the size of the primary particles obtained from spray dried droplets, whereby a low nicotinamide concentration in the aqueous solution leads to smaller primary particles, which, however, agglomerate more easily. Particle size is also influenced by the nozzle type, temperature and pressure in spray drying. The nozzle type and dimensions are chosen for obtaining droplets of a desired size. A high nozzle pressure provides smaller droplets, but the amount of fine dust in the device is increased. The temperature of the spray drying gas stream is adjusted not too high, such that residual water will remain bound in the particles for agglomeration. Also the gas flow rate influences drying, whereby an increased flow rate usually accelerates drying. The temperature of the internal fluidized bed is controlled such that a residual dryness is maintained for agglomeration, especially of small particles expelled from the fluidized bed. The pressure of the fluidizing gas stream is adjusted to maintain agglomerates of a desired size within the bed, whilst expelling smaller particles back into the drying chamber. The temperature of the external fluidized bed is adjusted for drying to a desired final moisture content.

Another important factor is residence time of the particles in the overall process. Overall, the particles should have a sufficiently high residence time for removal of residual moisture bound within the particles. It was found that allowing sufficient residence time in the overall process, especially by adaptation of the residence time within the fluidized bed(s), prevents caking. Caking is generally attributed to intercrystalline bridging by crystallization of the residual supersaturated solution at or near the surface of the nicotinamide granule. Probably the most common cause of this crystallization, and thus caking, is moisture migration from some region in the bulk of nicotinamide owing to the occurrence of temperature gradients and humidity differences. Without being bound to theory, it is speculated that especially a sufficient residence time in the additional drying step is advantageous not only for drying the particles, but also for formation of an advantageous internal structure, especially with respect to internal moisture distribution. The formation of uniform particles in the additional drying step is supported by the already uniform size distribution of the particles obtained from the fluidized spray dryer.

Another subject of the invention is a tablet, pellet or granule comprising nicotinamide, obtainable by direct compression of an inventive nicotinamide powder, or of a powder composition comprising an inventive nicotinamide powder. The composition may comprise common additives, especially excipient and lubricants, and/or other active ingredients, such as other vitamins. In preferred embodiments, the tablets, pellets or granules comprise at least 20%, 50%, 80%, 90%, 95% or 98% (w/w) nicotinamide. In a preferred embodiment, the tablets consist of nicotinamide.

### Brief description of the figures:

Fig. 1 schematically shows an exemplified device for carrying out a process of the invention.
Fig. 2 shows a scanning electron microscopy (SEM) image of nicotinamide crystallized according to a process in the art.
Fig. 3 shows a scanning electron microscopy (SEM) image of nicotinamide roll-compacted according to a process in the art.
Fig. 4 shows a scanning electron microscopy (SEM) image of nicotinamide powder of the invention produced by fluidized spray drying and subsequent fluidized bed drying.
Fig. 5 is a magnified scanning electron microscopy (SEM) image corresponding to Fig. 4.
Fig. 6 shows a plot of tablet force against compression force for tablets produced by direct compression of an inventive powder (standard convex tablet, 9 mm, 330 mg nicotinamide).

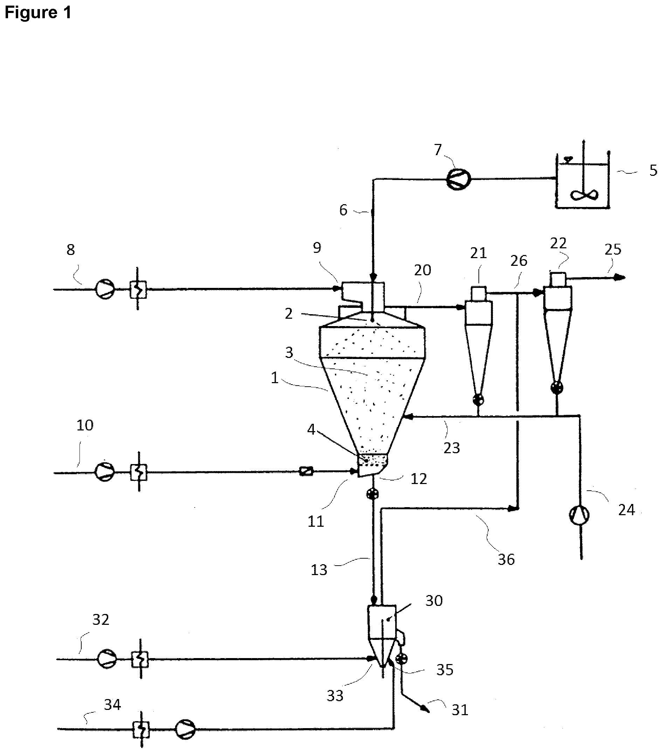

Fig. 1 illustrates in a schematic and exemplified form a device of the invention for carrying out a process of the invention. The device comprises a fluidized spray dryer 1 comprising spray drying means 2, such as nozzles, a drying chamber 3 and a fluidized bed 4. The spray drying means 2 are positioned above the central drying chamber 3. The fluidized bed 4 is positioned below the central drying chamber. Aqueous solution for feeding to the spray drying means 2 is heated in a tank 5. The aqueous solution is transferred to the spray drying means 2 through connections 6, such as tubes or hoses, whereby transfer may be mediated by a pump 7. A co-current gas stream 8 is fed into the fluidized spray dryer through inlet 9, such that the gas stream flows in the same direction as the spray dried droplets released from the spray drying means. A fluidizing gas stream 10 is fed into the fluidized spray dryer through inlet 11 at a position at the bottom of the device, such that a fluidized bed 4 is generated. Droplets released from nozzles 2 pass the drying chamber 3 and, after agglomeration to a sufficient size and weight, enter fluidized bed 4. Powder particles are removed from the fluidized bed through an overflow and withdrawn from the fluidized spray dryer through outlet 12. The product is transferred through connections 13 to an additional fluidized bed 30, which is external from the fluidized spray dryer. Alternatively, a series of multiple consecutive external fluidized beds may be used, for example a series of three fluidized beds for drying and one fluidized bed for cooling. The external fluidized bed is generated with a fluidizing gas stream 32 entering through inlet 33. The fluidized bed may comprise an additional cooling gas stream 34 entering through inlet 35. A nicotinamide powder of desired particle size and dryness is obtained through outlet 31. All gas streams for spray drying and operating fluidized beds may be dried air.

The device may comprise means for collecting and recycling fine dust. Fine dust may be collected at an upper section of the fluidized bed device through outlet 20 and led into accumulation means 21, 22. The accumulation means are cyclones. Two or more accumulation means may be combined in series, for example two cyclones. Multiple accumulation means are connected through connections 26. Fine dust accumulated in the accumulation means is re-introduced in pulverized form into the fluidized spray dryer through connections 23. The re-introduction may be supported by gas stream 24, for example as a pneumatic conveying system. Residual gas, from which fine dust was removed, can be diverted through exhaust connection 25, which may comprise a filter. In addition, fine dust emerging in the second fluidized bed 30 may be transferred into the recycling process through connection 36. Thus, it is ensured that overall fine dust is removed from the product, recycled and integrated into the product by agglomeration. The overall device comprises appropriate connections, such as tubes and hoses. Flow of gas streams and powders is supported by appropriate means, such as pneumatic conveying means and/or pumps, for example in connections 8, 10, 23, 24, 31, 24 and 7. The inventive process and nicotinamide powder solve the problems underlying the invention. The inventive process allows the production of a nicotinamide powder with an unexpected and novel combination of advantageous properties. Specifically, the nicotinamide powder has a high flowability and low tendency towards caking and lumping, whereas it is at the same time suitable for direct compression and pelletization. The powder does not comprise dust particles and thus does not release dust when processed. The properties are advantageous for packaging, transport and processing, for example in tableting machinery. The powder is highly homogeneous regarding particle size distribution and shape. Mixing with other components in a premix is homogeneous and the tendency to segregation is low. This is especially important for products such as vitamins, which are stored, transported and processed at a large scale. The overall production process can be carried out continuously and relatively convenient starting from an aqueous solution.

When fine dusts are recycled, the overall recovery of nicotinamide is nearly 100%, and thus waste is avoided. Further, the process can be carried out efficiently starting from aqueous solutions comprising about 80% (w/w) nicotinamide. Thus only about 20% (w/w) water have to be evaporated and thus the overall energy consumption can be kept low. The process is thus advantageous compared to other spraying processes, such as wet granulation, in which higher amounts of water are introduced into the process.

### Examples

### Methods:

Particle shape, mean particle size and particle size distribution were determined by sieve analysis (ALPINE air jet sieve) or LASER diffraction.

The angle of repose was determined according to DIN 53916.

Jenike flow function was determined with a Jenike shear tester as described by Jenike, A.W.: Storage and Flow of Solids, Bull. No. 123, Engin. Exp. Station, Univ. Utah, Salt Lake City (1970). The test is also described in "Standard Shear Testing Technique for Particulate Solids Using the Jenike Shear Cell", Institution of Chemical Engineers (European Federation of Chemical Engineering), Rugby UK, 1989. For determining caking, the Jenike flow function was determined after 24 h consolidation at equilibrium relative humidity (ERH; "relative humidity" over bulk solid in a closed vessel at equilibrium; the ERH of a substance is a state at which the substance neither gains nor loses moisture).

Bulk density was determined according to DIN 53912.

Tablet breaking force was determined as follows: Standard convex tablets of 9 mm diameter (band height 3 mm, curvature height 1 mm, overall height 5 mm) were produced from 330 mg nicotinamide with an eccentric press at a press force of 10 kN. Standard tablet shapes, such as standard convex, as used herein are those as defined by the American Pharmacist Association, 2010. After hardening for 24 h, tablet breaking force was determined in a diametral breakage test with a tablet tester (Schleuniger, DE). An average from 10 results was calculated.

### Example 1a: Fluidized spray drying

An inventive process was carried out with a device as shown in figure 1. A solution of 79% (w/w) nicotinamide in water is heated to 80 °C and is continuously conveyed with a high-pressure pump to a spray nozzle centered at the upper part of the spraying chamber of a fluidized spray dryer (265 kg/h, corresponding to 209 kg/h nicotinamide). The cylindrical upper part of the spraying chamber has a diameter of 1.7 m and a height of 1 m, the lower part is conical at an angle of 40°. The atomized nicotinamide solution is dried co-currently with hot air (125 °C, 1500 kg/h) entering the upper cylindrical part of the spraying drying chamber. Partly dried and agglomerated particles accumulate in the lower part of the drying chamber. The lower part below the conical portion contains an integrated fluidized bed, in which further agglomeration and separation from fine particles take place. The fluidized bed is operated by an air stream (860 kg/h) heated to 50 °C. Off-gas comprising fine nicotinamide dust is conducted out of the drying chamber at the top of the device. Fine dust from the off-gas is accumulated in an external cyclone. The fine dust is then reintroduced into the drying chamber, where it is agglomerated with sprayed particles. Solid particles reaching a certain size and weight are continuously discharged from the bottom of the fluidized bed and transferred out of the fluidized spray dryer by a rotary feeder. The solid product was subjected to an additional drying step in an external fluidized bed, which is fluidized by an airstream having a temperature of 19 °C.

### Example 1b: Fluidized spray drying

An inventive process was carried out according to example 1a. A subsequent drying step was carried out with an additional external fluid bed. The external fluidized bed 30 is constructed as cascade of 4 fluidized beds with 3 drying (heating) zone and 1 cooling zone. Solid particles reaching a certain size and weight are continuously discharged from the bottom of the fluidized bed and transferred out of the fluidized spray dryer by a rotary feeder. The solid product was subjected to an additional drying step cascade of 4 equal sized fluidized beds, which are fluidized by an airstream having a temperature of 90 °C in the first fluidized bed, 78 °C in the second and third fluidized bed, and 11 °C in the fourth fluidized bed. The total resident time in the cascade was 3 hours.

### Comparative Example 2: Cooling crystallization from water solution

Nicotinamide was dissolved at 80 °C in water to obtain a 50% (w/w) solution. After cooling to 20 °C, the needle shaped crystals were separated by centrifugation. After washing and drying, the solid powder is used for further product assessment.

### Comparative Example 3: Flaking

A melt of nicotinamide was poured out on a cold metal plate and cristallized. The solid product was crushed (Frewitt sieve granulator) and screened (sieving). A sieve fraction with particle size 200 to 800 µm was used for further product assessment.

### Comparative Example 4: Prilling of melted nicotinamide

Nicotinamide (1000 g) was molten in a 2 l pressure vessel at 160 to 180 °C for about 5 h. The melt temperature was maintained at 150 °C. The melt was sprayed into a spraying chamber (11 kg/h) with a pressure atomizer (scattering angle 30°, nozzle diameter 0.5 mm, 4 bar). Spraying pressure was adjusted with nitrogen gas, such that a fine spray mist was generated. A counter-current gaseous stream of cold nitrogen (5 °C) was led into the spray chamber (60 l/min). The nitrogen gas had been cooled before with liquid nitrogen. The average temperature in the spraying chamber was about -5 °C to 5 °C.

### Comparative Example 5: Roll compaction

A solid product produced by cooling crystallization according to example 2 was compacted with a roller compactor (pressure 95 kN, gap width 4 mm, 60 kg/h, recycling of oversized and undersized grains), crushed with a Frewitt sieve granulator (sieve 1 mm, round wire) and sieved for undersized grains (mesh size 180 µm, 0.72 m²) and oversized grains (630 µm, 0.36 m²). A sieve fraction with an average particle size of 400 µm was obtained for further product assessment.

### Comparative Example 6: Fluidized bed spray granulation

700 g wet crystallized nicotinamide (from centrifugation in example 2) was filled into a fluidized bed spray granulator. 350 g of a warm (60 °C) solution of 70% nicotinamide in water is continuously sprayed (13 g/min) with a spray nozzle (two component jet) into the fluidized bed. Fluidizing and drying is mediated by hot air (inlet temperature 95 °C, outlet temperature 30 °C). After addition of granulation liquid, the final drying was continued until the outlet temperature reached 60 °C.

### Comparison of results

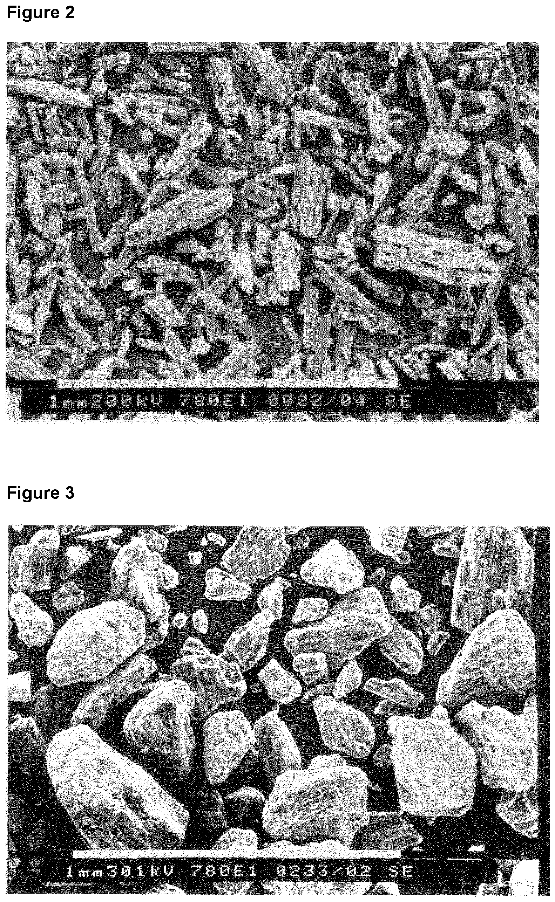

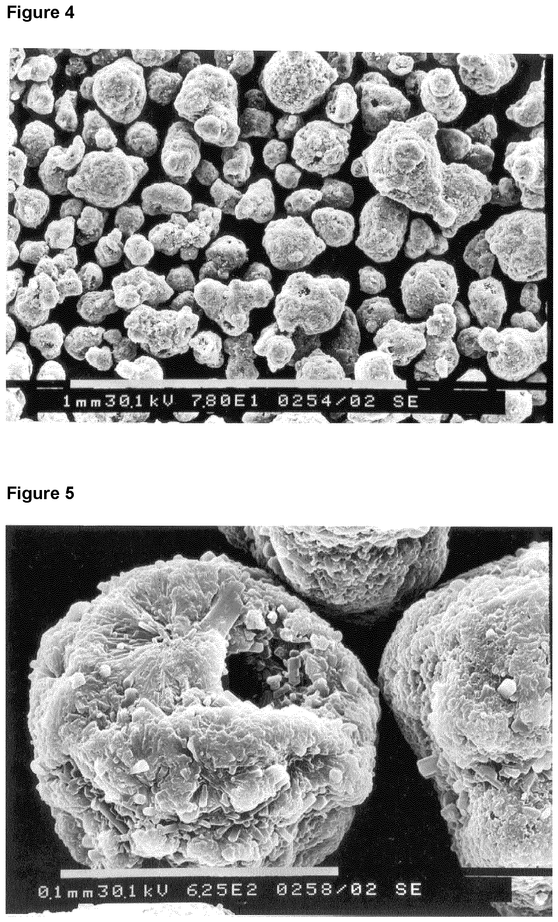

Images created by Scanning Electron microscopy (SEM) of the inventive product of example 1 are shown in figures 4 and 5. The particles have a uniform size distribution and approximately spherical shapes. SEM images of comparative products are shown in figure 2 (crystallized, example 2) and figure 5 (roll-compressed, example 3). The respective particles have heterogeneous size distributions and irregular shapes.

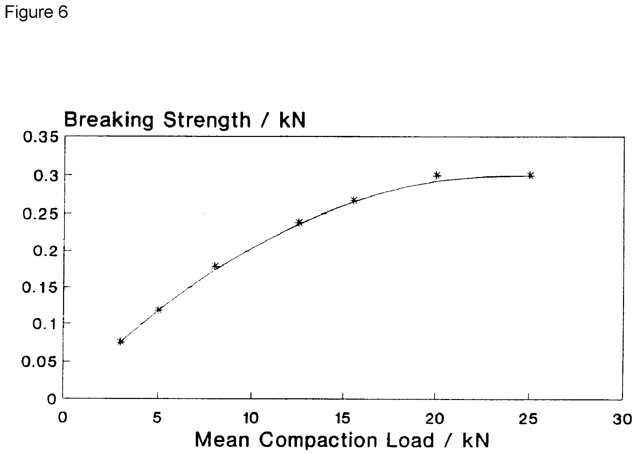

Properties of the products were determined by the test methods described above. The results are summarized in table 1 below. In general, a tablet breaking force of at least 150 N is considered acceptable for direct compression, whereas a tablet breaking force below 100 N is insufficient. Flowability of the powders is determined by measuring the angle of repose and determining Jenike flow function. A low angle of repose and a Jenike flow function of about 10 are indicative of good flowability. The results show, that the inventive powder prepared according to example 1 combines excellent flowability with excellent direct compressibility. In contrast, powders prepared according to other processes do not combine good flowability with sufficient direct compressibility. Caking of the powders is indicated by the Jenike flow function measured after 24 h consolidation. The inventive powder of example 1a still has good flow properties after 24 h and thus a relatively low tendency to caking. When conducting an additional drying step with an external fluidized bed, the tendency towards caking can be reduced even further. Figure 6 shows tablet breaking force against compression force of a tablet produced by direct compression of an inventive powder (standard convex tablet, 9 mm, 330 mg nicotinamide). Tablet breaking forces up to about 300 N can be obtained by increasing the compression force moderately. Overall, the inventive powder combines good flowability, low tendency to caking and good direct compressibility.

In addition, the inventive powders have relatively homogeneous particle size distribution as well as relatively uniform particle sizes close to spherical. The average particle size of the product of example 1b is about 180 µm. The amount of particles with a diameter <63 µm was only 3.2% and the amount of particles <100 µm was only 18% (ALPINE air jet sieve), indicating a narrow particle size distribution. The uniform size and structure and the almost spherical shape are illustrated by figures 4 and 5. Figure 2 shows a comparative crystallized powder and figure 3 shows a comparative roll-compacted powder. The shapes are irregular and the size distribution is wide.

**Table 1: Comparison of product properties**

| **Example No.** | **1** (inventive) | **1b** (inventive) | **2** (comparative) | **3** (comparative) | **4** (comparative) | **5** (comparative) | **6** (comparative) |
|---|---|---|---|---|---|---|---|
| Process | fluidized bed spray drying | fluidized bed spray drying | crystallization | flaking/sieving | melt spray drying (prilling) | roll compaction/ sieving | fluidized bed spray granulation |
| Particle shape | approximately spherical | approximately spherical | needles | irregular | spherical | irregular | approximately spherical |
| Mean particle size [µm] | 175 | 180 | 300 | 380 | 230 | 400 | 205 |
| Particle size distribution | sharp | sharp | broad | moderate | very sharp | moderate | moderate-sharp |
| Angle of repose[°] | 35 | 34 | 55 | 39 | 30 | 37 | 39 |
| Jenike flow function (stress/ unconfined yield stress) | 45 | 51 | 2 | 15 | 48 | 29 | 21 |
| Jenike flow function (consolidation time 24 h, 20 °C equilibrium relative humidity) | 9 | 12 | <1 | 7 | - | 4 | 7 |
| Bulk density [g/cm³] | 0.51 | 0.51 | 0.51 | 0.72 | 0.70 | 0.71 | 0.53 |
| Tablet breaking force [N] | 200 | 195 | 45 | 32 | 25 | 126 | 90 |

## Claims

1. A process for the preparation of a nicotinamide powder, comprising the steps of
(a) providing an aqueous solution of nicotinamide,
(b) spray drying the solution and
(c) fluidizing the spray dried nicotinamide in a fluidized bed (4),
wherein steps (b) and (c) are carried out in a fluidized spray dryer (1).

2. The process of claim 1, wherein the fluidized spray dryer comprises
(A) a central drying chamber (3),
(B) spray drying means (2), positioned above the central drying chamber (3),
(C) a fluidized bed (4), positioned below the central drying chamber, and
(D) means for withdrawing the nicotinamide powder, positioned below the central drying chamber.

3. The process of at least one of the preceding claims, wherein the spray drying step (b) is conducted with a co-current gas stream (8) having an inlet temperature between 100 °C and 180 °C and/or wherein the fluidized bed comprises a fluidizing gas stream (10) having a temperature between 30 °C and 90 °C.

4. The process of at least one of the preceding claims, wherein the aqueous solution comprises 50% to 90% (w/w) nicotinamide.

5. The process of at least one of claims 1 to 3, wherein nicotinamide fine dust is withdrawn from the fluidized spray dryer (1), the nicotinamide fine dust is then accumulated in accumulation means (21, 22), the accumulation means preferably comprising at least one cyclone and/or filter, and thus accumulated nicotinamide fine dust is reintroduced into the fluidized spray dryer.

6. The process of at least one of claims 1 to 3, whereby the nicotinamide powder obtained from the fluidized spray dryer (1) is subjected to an additional drying step.

7. The process of at least one of the preceding claims, comprising an additional drying step in an additional fluidized bed (30), wherein the temperature of the additional fluidized bed (30) is between 60 °C and 100 °C.

8. The process of at least one of the preceding claims, wherein the water content of the nicotinamide powder obtained from the fluidized spray dryer (1) is between 0.05% and 0.3% (w/w) and/or wherein the moisture content of the nicotinamide powder obtained after the additional drying step is between 0.01% and 0.07% (w/w).

9. Nicotinamide powder, having
- an average particle diameter between 50 µm and 500 µm,
- an angle of repose below 40°, and
- a tablet breaking force above 150 N, preferably above 175 N, after direct compression of 330 mg nicotinamide into a standard convex tablet, diameter 9 mm, with a press force of 10 kN, and/or a tensile strength of at least 2.5 N/mm², more preferably at least 3.0 N/mm², for a tablet obtained by direct compression with a compression pressure of 157 N/mm².

10. A tablet, pellet or granule comprising nicotinamide, obtained by direct compression of the nicotinamide powder of claim 9 or of a powder composition comprising the nicotinamide powder of claim 9.

## Patentansprüche

1. Verfahren für die Herstellung eines Nicotinamidpulvers, umfassend die Schritte des
(a) Bereitstellens einer wässrigen Lösung von Nicotinamid,
(b) Sprühtrocknens der Lösung und
(c) Fluidisierens des sprühgetrockneten Nicotinamids in einem Wirbelbett (4),
wobei die Schritte (b) und (c) in einem Wirbelsprühtrockner (1) ausgeführt werden.

2. Verfahren nach Anspruch 1, wobei der Wirbelsprühtrockner Folgendes umfasst
(A) eine mittige Trockenkammer (3),
(B) ein Sprühtrocknungsmittel (2), das über der mittigen Trockenkammer (3) positioniert ist,
(C) ein Wirbelbett (4), das unter der mittigen Trockenkammer positioniert ist, und
(D) ein Mittel zum Abziehen des Nicotinamidpulvers, das unter der mittigen Trockenkammer positioniert ist.

3. Verfahren nach mindestens einem der vorhergehenden Ansprüche, wobei der Sprühtrocknungsschritt (b) mit einem gleichzeitig strömenden Gasstrom (8) ausgeführt wird, der eine Einlasstemperatur zwischen 100 °C und 180 °C aufweist und/oder wobei das Wirbelbett einen fluidisierten Gasstrom (10) umfasst, der eine Temperatur zwischen 30 °C und 90 °C aufweist.

4. Verfahren nach mindestens einem der vorhergehenden Ansprüche, wobei die wässrige Lösung 50 % bis 90 % (Gew./Gew.) Nicotinamid umfasst.

5. Verfahren nach mindestens einem der Ansprüche 1 bis 3, wobei Nicotinamidfeinstaub aus dem Wirbelsprühtrockner (1) abgezogen wird, der Nicotinamidfeinstaub dann in Ansammlungsmitteln (21, 22) angesammelt wird, wobei die Ansammlungsmittel bevorzugt mindestens einen Zyklon und/oder ein Filter umfassen und so angesammelter Nicotinamidfeinstaub erneut in den Wirbelsprühtrockner eingeführt wird.

6. Verfahren nach mindestens einem der Ansprüche 1 bis 3, wobei das Nicotinamidpulver, das aus dem Wirbelsprühtrockner (1) erhalten worden ist, einem zusätzlichen Trocknungsschritt unterworfen wird.

7. Verfahren nach mindestens einem der vorhergehenden Ansprüche, umfassend einen zusätzlichen Trocknungsschritt in einem zusätzlichen Wirbelbett (30), wobei die Temperatur des zusätzlichen Wirbelbetts (30) bei zwischen 60 °C und 100 °C liegt.

8. Verfahren nach mindestens einem der vorhergehenden Ansprüche, wobei der Wassergehalt des Nicotinamidpulvers, das aus dem Wirbelsprühtrockner (1) erhalten worden ist, zwischen 0,05 % und 0,3 % (Gew./Gew.) beträgt und/oder wobei der Feuchtigkeitsgehalt des Nicotinamidpulvers, das nach dem zusätzlichen Trocknungsschritt erhalten worden ist, zwischen 0,01 % und 0,07 % (Gew./Gew.) beträgt.

9. Nicotinamidpulver, das Folgendes aufweist
- einen durchschnittlichen Teilchendurchmesser zwischen 50 µm und 500 µm,
- einen Schüttwinkel unter 40°, und
- eine Tablettenbrechkraft von über 150 N, bevorzugt über 175 N, nach direktem Komprimieren von 330 mg Nicotinamid in eine normale konvexe Tablette, Durchmesser 9 mm, mit einer Presskraft von 10 kN und/oder einer Zugfestigkeit von mindestens 2,5 N/mm², noch bevorzugter mindestens 3,0 N/mm², bei einer Tablette, die durch direktes Komprimieren mit einem Komprimierdruck von 157 N/mm² erhalten worden ist.

10. Tablette, Kügelchen oder Körnchen umfassend Nicotinamid, die/das durch direktes Komprimieren des Nicotinamidpulvers nach Anspruch 9 oder einer Pulverzusammensetzung, die das Nicotinamidpulver nach Anspruch 9 umfasst, erhalten wird.

## Revendications

1. Procédé de préparation d'une poudre de nicotinamide, comprenant les étapes qui consistent à :
(a) fournir une solution aqueuse de nicotinamide,
(b) sécher la solution par atomisation, et
(c) fluidiser le nicotinamide séché par atomisation dans un lit fluidisé (4),
dans lequel les étapes (b) et (c) sont exécutées dans un séchoir par atomisation à lit fluidisé (1) .

2. Procédé selon la revendication 1, dans lequel le séchoir par atomisation à lit fluidisé comprend :
(A) une chambre de séchage centrale (3),
(B) un moyen de séchage par atomisation (2), positionné au-dessus de la chambre de séchage centrale (3),
(C) un lit fluidisé (4), positionné en dessous de la chambre de séchage centrale, et
(D) un moyen de retrait de la poudre de nicotinamide, positionné en dessous de la chambre de séchage centrale.

3. Procédé selon au moins l'une des revendications précédentes, dans lequel l'étape de séchage par atomisation (b) est conduite avec un flux de gaz à cocourant (8) ayant une température d'entrée de 100 °C à 180 °C et/ou dans lequel le lit fluidisé comprend un flux de gaz fluidisé (10) ayant une température de 30 °C à 90 °C.

4. Procédé selon au moins l'une des revendications précédentes, dans lequel la solution aqueuse comprend 50 % à 90 % (p/p) de nicotinamide.

5. Procédé selon au moins l'une des revendications 1 à 3, dans lequel de la poussière fine de nicotinamide est retirée du séchoir par atomisation à lit fluidisé (1), la poussière fine de nicotinamide s'accumule ensuite dans un moyen d'accumulation (21, 22), le moyen d'accumulation comprenant préférablement au moins un cyclone et/ou un filtre, et la poussière fine de nicotinamide qui s'est ainsi accumulée est réintroduite dans le séchoir par atomisation à lit fluidisé.

6. Procédé selon au moins l'une des revendications 1 à 3, dans lequel la poudre de nicotinamide obtenue à partir du séchoir par atomisation à lit fluidisé (1) est soumise à une étape de séchage additionnelle.

7. Procédé selon au moins l'une des revendications précédentes, comprenant une étape additionnelle de séchage dans un lit fluidisé additionnel (30), dans lequel la température du lit fluidisé additionnel (30) est de 60 °C à 100 °C.

8. Procédé selon au moins l'une des revendications précédentes, dans lequel la teneur en eau de la poudre de nicotinamide obtenue à partir du séchoir par atomisation à lit fluidisé (1) est de 0,05 % à 0,3 % (p/p) et/ou dans lequel la teneur en humidité de la poudre de nicotinamide obtenue après l'étape de séchage additionnelle est de 0,01 à 0,07 % (p/p).

9. Poudre de nicotinamide, ayant :
- un diamètre de particule moyen de 50 µm à 500 µm,
- un angle de talus d'éboulement inférieur à 40 °, et
- une force de rupture de comprimé supérieure à 150 N, préférablement supérieure à 175 N, après compression directe de 330 mg de nicotinamide pour produire un comprimé convexe standard, de 9 mm de diamètre, avec une force de compression de 10 kN, et/ou une résistance à la traction d'au moins 2,5 N/mm², plus préférablement d'au moins 3,0 N/mm², pour un comprimé obtenu par compression directe avec une pression de compression de 157 N/mm².

10. Comprimé, pastille ou granulé comprenant du nicotinamide, obtenu(e) par compression directe de la poudre de nicotinamide selon la revendication 9 ou d'une composition de poudre comprenant la poudre de nicotinamide selon la revendication 9.
